# EUROPÄISCHE PATENTSCHRIFT

(11) **EP 2 424 431 B1**
(45) Veröffentlichungstag und Bekanntmachung des Hinweises auf die Patenterteilung: **30.08.2017**
(21) Anmeldenummer: 10716763.7
(22) Anmeldetag: 15.04.2010
(51) Int. Cl.: A61B 5/00

(54) **MEDIZINISCHES DIAGNOSEGERÄT SOWIE VERFAHREN ZUR DIAGNOSE**
MEDICAL DIAGNOSTIC DEVICE AND METHOD FOR DIAGNOSING
APPAREIL DE DIAGNOSTIC MÉDICAL ET PROCÉDÉ DE DIAGNOSTIC

(30) Priorität: 30.04.2009 DE 102009019242
(43) Veröffentlichungstag der Anmeldung: 07.03.2012
(73) Patentinhaber: Technische Universität Carolo-Wilhelmina zu Braunschweig, 38106 Braunschweig (DE)
(72) Erfinder: SCHILLING, Meinhard, 38302 Wolfenbüttel (DE); OEHLER, Martin, 38108 Braunschweig (DE)
(74) Vertreter: Günther, Constantin
(86) Internationale Anmeldenummer: PCT/EP2010/002312
(87) Internationale Veröffentlichungsnummer: WO 2010/124794

(56) Entgegenhaltungen:
- EP-A1- 1 800 599
- WO-A1-2008/032234
- DE-A1-102007 007 563
- US-A1- 2008 097 232
- US-A1- 2009 234 242

## Beschreibung

Die Erfindung betrifft ein medizinisches Diagnosegerät gemäß dem Oberbegriff des Patentanspruchs 1 sowie ein entsprechendes Verfahren zur medizinischen Diagnose.

Aus der DE 10 2004 063 249 A1 ist ein Sensorsystem zur kapazitiven Messung elektromagnetischer Signale biologischen Ursprungs bekannt. Mittels der dort erläuterten kapazitiven Elektroden kann auf neuartige Weise z. B. ein Elektrokardiogramm (EKG) oder ein Elektroenzephalogramm (EEG) aufgenommen werden. Die dort vorgestellten Elektroden lassen sich kostengünstig fertigen, so dass ein entsprechendes medizinisches Diagnosegerät mit einer Mehrzahl solcher Elektroden als Sensoren ausgestattet werden kann. Die Mehrzahl der Elektroden kann beispielsweise matrixförmig angeordnet werden und so eine Sensormatrix bilden. Hierdurch können die bei der Erstellung von EKGs erforderlichen Messungen an verschiedenen Körperstellen auf einfache Weise vorgenommen werden. Insbesondere erübrigt sich ein Befestigen einer Vielzahl von Einzelelektroden am Körper, wie von konventionellen EKG-Systemen mit Klebe- oder Saugelektroden her bekannt. Die neuartigen kapazitiven Elektroden erlauben insbesondere auch eine EKG-Aufnahme durch Textilien hindurch, so dass ein Patient nicht entkleidet werden muss.

Aus der EP 1 800 599 A1 ist ein Sensor zur Messung elektrischer Variablen an einer Oberfläche eines menschlichen oder tierischen Körpers bekannt, der drei oder mehr Elektroden in einer regelmäßigen geometrischen Anordnung aufweist. Der Sensor weist außerdem einen Halter zum Zusammenhalten der Elektroden auf.

Aus der DE 10 2007 007 563 A1 geht ein Verfahren zur Ermittlung der kardialen Reizleitung und eine zugehörige medizinische Einrichtung hervor, bei denen mehrere verteilte Elektrokardiogramm-Elektroden vorgesehen sind.

Aus der US 2008/0312522 A1 ist ein medizinisches Diagnosegerät mit einer Sensormatrix bekannt, die eine Mehrzahl von in bestimmter Weise verteilten Sensoren aufweist und zur Erfassung von elektrischen Spannungssignalen an einer Mehrzahl von Stellen an der Körperoberfläche eines Menschen ausgebildet ist.

Der Erfindung liegt die Aufgabe zugrunde, ein medizinisches Diagnosegerät mit einer derartigen Sensormatrix anzugeben, das gegenüber bekannten EKG-Systemen einen größeren Einsatzbereich und verbesserte Diagnosemöglichkeiten erlaubt. Es ist außerdem Aufgabe der Erfindung, hierfür ein geeignetes Diagnoseverfahren anzugeben.

Diese Aufgabe wird durch die Erfindung gemäß den Ansprüchen 1 und 13 gelöst. Die Unteransprüche geben vorteilhafte Weiterbildungen der Erfindung an. Die Erfinder haben erkannt, dass ein medizinisches Diagnosegerät mit einer Sensormatrix, d. h. einer Mehrzahl von in bestimmter Weise verteilten Sensoren, neben der bekannten klassischen Darstellung von EKG-Messungen in Form von Zeitdiagrammen auch ortsaufgelöste bildliche Darstellungen vorteilhaft erlaubt, z. B. in Form von zweidimensionalen Bildinformationen. Die aus den mittels der Sensormatrix erfassten Spannungssignalen gewonnene Bildinformation kann gemäß der Erfindung vorteilhaft um eine Positionsinformation ergänzt werden. Die Positionsinformation gibt die Position der Sensormatrix relativ zum Körper an. Dies erlaubt eine einfache und schnelle Erkennbarkeit von zugehörigen Körperbereichen und erleichtert und beschleunigt damit die Auswertung durch einen Arzt und die Erstellung der medizinischen Diagnose. Zudem wird die Reproduzierbarkeit der Messungen verbessert, da die Positionsinformation eine schnelle Orientierung der die Bildinformation auswertenden Person hinsichtlich der Bildinhalte ermöglicht.

Es ist insbesondere möglich, ein Bild der Anatomie des untersuchten Körpers gleichzeitig mit der funktionellen Bildgebung, d. h. der über die Sensormatrix erzeugten Bildinformationen, zu erzeugen. Hierdurch kann die Bildinformation relativ zur Lage der anatomischen Situation im untersuchten Körperteil dargestellt werden. Mittels der Erfindung kann vorteilhaft das funktionelle Bild am anatomischen Bild orientiert und unabhängig von der Position und Lage des Diagnosegeräts an der Anatomie ausgerichtet dargestellt werden. So kann beispielsweise neben einem zweidimensional dargestellten und damit ortsaufgelösten EKG als weitere Bildinformation auch die Lage der Lungen oder der Rippen dargestellt werden. Damit ist die Position des Diagnosegeräts relativ zum Brustkorb unwichtig, weil das ortsaufgelöste EKG nicht mehr relativ zur Sensormatrix dargestellt werden muss, sondern automatisch relativ zur Anatomie des untersuchten Körpers dargestellt werden kann.

Gemäß einer vorteilhaften Weiterbildung der Erfindung ist das Diagnosegerät zur Erfassung eines Elektrokardiogramms oder eines Elektroenzephalogramms eingerichtet. Hierdurch ist das erfindungsgemäße Diagnosegerät besonders universell einsetzbar. In der Ausführung zur Erfassung eines Elektroenzephalogramms wird die Sensormatrix an einer am Kopf des zu untersuchenden Menschen oder Tieres zu befestigenden Einheit, z. B. einem Helm, angebracht.

Die Position der Sensormatrix relativ zum Körper des Menschen oder Tieres kann vorteilhaft insbesondere nach dem Prinzip der Impedanztomografie mit elektrischen und/oder magnetischen Signalen durchgeführt werden.

Gemäß einer vorteilhaften Weiterbildung der Erfindung ist das Diagnosegerät zur Erfassung eines elektromagnetischen Umgebungsfelds und zur Auswertung des erfassten Felds zur Bestimmung der Position der Sensormatrix relativ zum Körper des Menschen oder Tieres eingerichtet. Als Umgebungsfeld kommt im Prinzip jedes elektrische, magnetische oder elektromagnetische Feld in Frage, das ohnehin in der Umgebung vorhanden ist, wie z. B. Störstrahlung von Haushaltsgeräten. Es kann hierbei ein beliebiges externes Feld erfasst werden, z. B. das 50 Hertz-Feld der 220 Volt-Wechselspannungsversorgung.

Insbesondere kann als Umgebungsfeld das von Rundfunksendern ausgestrahlte Rundfunksignal verwendet werden, da es eine relativ hohe Feldstärke aufweist und aus einer im Verhältnis zur Größe des Diagnosegeräts relativ großen Entfernung abgestrahlt wird. Aufgrund der großen Entfernung ist das zur Positionsbestimmung erfasste Feld im Wesentlichen invariant gegenüber geringfügigen Positionsänderungen des zu untersuchenden Körpers oder des Diagnosegeräts. Damit ist über ein solches Umgebungsfeld eine relativ gut reproduzierbare und präzise Positionsbestimmung der Position der Sensormatrix relativ zum Körper des Menschen oder Tieres möglich.

Vorteilhaft werden zur Erfassung des Signals, das die Position der Sensormatrix relativ zum Körper des Menschen oder Tieres repräsentiert, die selben Sensoren der Sensormatrix verwendet wie für die Erfassung der elektrischen Spannungssignale an der Körperoberfläche des Menschen oder Tieres. Somit ist es vorteilhaft möglich, ohne zusätzliche Sensoren auszukommen, wodurch die Erfindung relativ kostengünstig und effizient praktisch realisiert werden kann. Insbesondere ist auch keine zusätzliche Auswerteelektronik für weitere Sensorsignale erforderlich.

In einer vorteilhaften Ausgestaltung kann das erfindungsgemäße Diagnosegerät jedoch auch einen weiteren Sensor zur Erfassung des die Position der Sensormatrix relativ zum Körper repräsentierenden Signals aufweisen. Der weitere Sensor kann zur Erfassung eines magnetischen Felds, eines elektrischen Felds, eines elektromagnetischen Felds, eines Schallfelds oder von Röntgenstrahlung ausgebildet sein. Dementsprechend legt der Fachmann das erforderliche Sensor-Funktionsprinzip aus. Der weitere Sensor kann beispielsweise an einer oder, im Falle mehrerer weiterer Sensoren, an verschiedenen definierten Positionen im Bereich der Sensormatrix an dem Diagnosegerät angeordnet sein. Die Verwendung eines separaten weiteren Sensors zur Erfassung des Positionssignals hat den Vorteil, dass unterschiedliche Sensor- Funktionsprinzipien verwendet werden können. So kann beispielsweise die Sensormatrix aus kapazitiven Sensoren gemäß der DE 10 2004 063 249 A1 aufgebaut sein. Der weitere Sensor kann z. B. ein Schallsensor, z. B. ein Mikrofon, sein. Hierdurch sind unterschiedliche physikalische Größen als Positionssignal verwertbar. Auch eine Kombination verschiedener Sensorprinzipien zur Auswertung verschiedener Positionssignale ist vorteilhaft.

Gemäß einer vorteilhaften Weiterbildung der Erfindung ist ein Signalkoppler zur Einkopplung eines Positionssignals in den Körper des Menschen oder Tieres vorgesehen. Der Signalkoppler kann mit dem Diagnosegerät verbunden ausgeführt oder als separates Gerät ausgeführt sein. Vorteilhaft ist der Signalkoppler an einer definierten Position bezüglich des zu untersuchenden Körpers anzuordnen. Das eingekoppelte Signal verteilt sich im Körper und wird von dem Diagnosegerät wiederum erfasst und ausgewertet.

So kann der Signalkoppler beispielsweise für die Untersuchung von Menschen etwa immer unterhalb der linken Achsel anzuordnen sein. Hierdurch kann eine definierte Positionszuordnung der Bildinformationen auch bei uneinheitlichen oder nicht auswertbaren Umgebungsfeldern sicher und gut reproduzierbar vorgenommen werden. Ein separater Signalkoppler zur Einkopplung des Positionssignals hat den weiteren Vorteil, dass ein bestimmtes gewünschtes physikalisches Signal eingekoppelt werden kann, wie z. B. ein Ultraschallsignal oder ein Röntgensignal. Somit können für das Positionssignal auch physikalische Signalarten verwendet werden, die als Umgebungsfelder in der Regel nicht verfügbar oder nicht geeignet sind.

Der Signalkoppler ist vorteilhaft zur Einkopplung eines magnetischen Felds, eines elektrischen Felds, eines elektromagnetischen Felds, eines Schallfelds oder von Röntgenstrahlung ausgebildet. Im Fall der Ausbildung als Schallquelle kann der Signalkoppler beispielsweise als Ultraschallquelle ausgebildet sein. Die Ultraschallquelle kann z. B. vorteilhaft in das Diagnosegerät integriert sein und im Bereich der Sensormatrix angeordnet sein. Vorteilhaft kann neben Ultraschall auch jede andere Schallart zur Einkopplung in den Körper verwendet werden, wie z. B. Musiksignale.

Vorteilhaft ist der weitere Sensor zur Erfassung des die Position der Sensormatrix relativ zum Körper repräsentierenden Signals für die gleiche physikalische Signalart, z. B. Ultraschall oder Röntgenstrahlung, ausgebildet wie der Signalkoppler.

Gemäß einer vorteilhaften Weiterbildung der Erfindung ist eine Anzeigeeinrichtung vorgesehen. Das Diagnosegerät ist dazu eingerichtet, eine Mehrzahl einzelner über die Sensormatrix bestimmter Bildinformationen anhand ihrer Positionsinformationen miteinander zu korrelieren. Mittels einer solchen Korrelation einer Mehrzahl von Bildinformationen kann beispielsweise die Größe der Bilddarstellung und/oder die Auflösung der Darstellung erhöht werden. Zur Erhöhung der Bildgröße wird vorteilhaft eine Mehrzahl von Bildinformationen aneinander gesetzt dargestellt. Hierbei werden die Bildinformationen nebeneinander und übereinander kombiniert, so dass ein vergrößerter Bilddarstellungsbereich erzeugt wird. Zur Erhöhung der Auflösung der Darstellung wird vorteilhaft eine Mehrzahl von Bildinformationen überlagert dargestellt. Die Überlagerung kann dabei z.B. derart vorgenommen werden, dass die Bildinformationen mit einem geringfügigen Versatz zueinander, der in der Größenordnung eines Bruchteils einer Pixelinformation einer ursprünglich erfassten Bildinformation liegt, überlagert werden. Durch entsprechende Überlagerung einer größeren Anzahl von Bildinformationen und geringfügiger Verschiebung in beiden Koordinatenrichtungen kann eine Vervielfachung der Auflösung der Darstellung erzielt werden.

Als Bildinformation wird neben einer Einzelbild-Information auch eine Bildfolge im Sinne eines Films verstanden. Da das Diagnosegerät vorteilhaft zur Aufnahme von eines EKGs genutzt werden kann, ist mit einem Abspielen der erfindungsgemäßen ortsaufgelösten Bildinformation zeitlich nacheinander nach Art eines Films wiederum eine EKG-Darstellung über die Zeit möglich, die mit EKG-Zeitverläufen konventionellen Systeme verglichen werden kann. Vorteilhaft ist das Diagnosegerät dazu eingerichtet, den Film mit einer vom Benutzer auswählbaren Geschwindigkeit abzuspielen. Insbesondere ist ein Abspielen in zeitlich gedehnter Darstellung (Zeitlupe) vorteilhaft, um eine Diagnose aufgrund der Bildinformation zu erstellen.

In einer vorteilhaften Ausgestaltung der Erfindung ist das medizinische Diagnosegerät als von einem Menschen tragbares Gerät ausgebildet und damit sozusagen ein portables Gerät. Vorteilhaft kann das Gerät damit in mobilen medizinischen Einsätzen mitgeführt werden, wie z. B. in Krankenwagen oder von Notärzten.

Die Erfindung ermöglicht ein vorteilhaftes Verfahren zur medizinischen Diagnose mit den Schritten
- Erfassen von elektrischen Spannungssignalen an einer Mehrzahl von Stellen an der Körperoberfläche eines Menschen oder Tieres mittels einer Sensormatrix,
- Erfassen eines die Position der Sensormatrix relativ zum Körper des Menschen oder Tieres repräsentierenden Signals,
- Bestimmung einer Positionsinformation der Position der Sensormatrix relativ zum Körper,
- Bestimmung einer ortsaufgelösten Bildinformation aus den Spannungssignalen und
- Zuordnung der Positionsinformation zu der Bildinformation.

Das Verfahren erlaubt insbesondere eine Verwendung der zuvor erläuterten Funktionen des Diagnosegeräts.

Die Erfindung wird nachfolgend anhand von Ausführungsbeispielen unter Verwendung von Zeichnungen näher erläutert.

Es zeigen
- Fig. 1 bis 3: ein Diagnosegerät in verschiedenen Ansichten und
- Fig. 4 und 5: weitere Ausführungsformen des Diagnosegeräts und
- Fig. 6: ein EKG-Diagramm und
- Fig. 7: ein Mehrfach-EKG-Diagramm und
- Fig. 8: ein zweidimensional ortsaufgelöstes EKG-Diagramm.

In den Figuren werden für einander entsprechende Elemente gleiche Bezugszeichen verwendet.

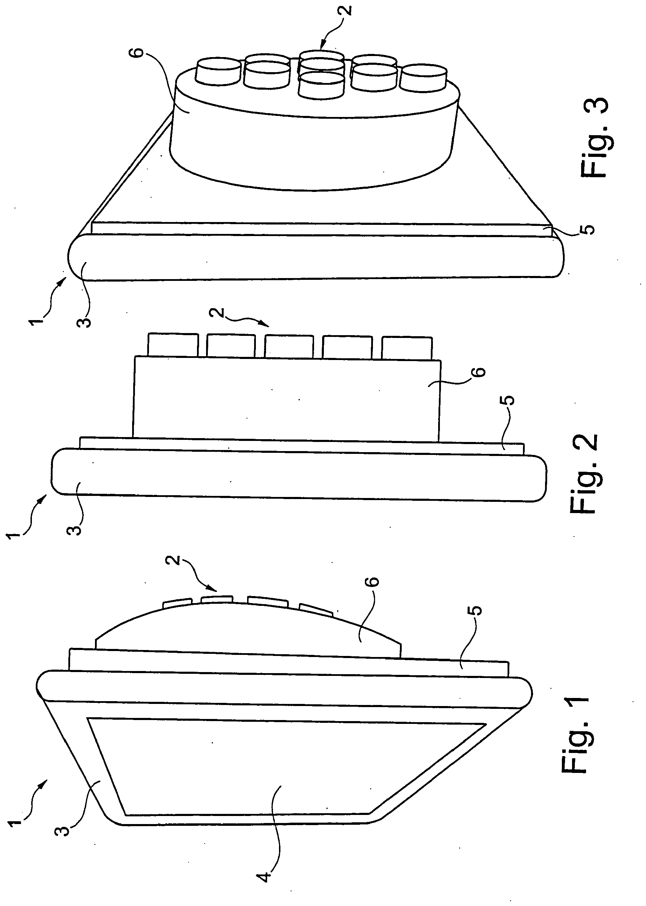

In der Fig. 1 ist das erfindungsgemäße Diagnosegerät in perspektivischer Darstellung von links vorne dargestellt. Fig. 2 zeigt eine Seitenansicht. Fig. 3 zeigt das Diagnosegerät in perspektivischer Darstellung von links hinten. Das Diagnosegerät 1 weist eine Computereinrichtung 3 mit einer Anzeigeeinrichtung 4 auf. Die Computereinrichtung 3 kann beispielsweise als Tablet-PC ausgebildet sein. Vorteilhaft kann auch ein Laptop oder Notebook verwendet werden. Hierdurch ist das Diagnosegerät relativ handlich und gut transportabel. Auf der Rückseite der Computereinrichtung 3 ist über eine Montageplatte 5 ein Gehäuse 6 angeordnet. Aus einer Rückseite des Gehäuses 6 ragen nach einem vorgegebenen regelmäßigen Muster angeordnete Sensoren heraus, die eine Sensormatrix 2 bilden. Die Sensoren sind beispielsweise als kapazitive Sensoren gemäß DE 10 2004 063 249 A1 ausgebildet. In einer vorteilhaften Ausführung sind die Sensoren gefedert in dem Gehäuse 6 angeordnet, so dass sie beim Aufsetzen auf den Körper eines Menschen oder Tieres nachgiebig sind und sich der Körperform in gewissem Ausmaß anpassen können. An ihrer zum untersuchenden Körper hin gerichteten Seite weisen die Sensoren jeweils eine Isolierung auf, die einen galvanischen Kontakt mit der Körperoberfläche verhindert.

In dem Gehäuse 6 ist ferner eine Auswerteeinrichtung in Form einer elektronischen Schaltung vorgesehen, die die von der Sensormatrix erzeugten Spannungssignale erfasst, verarbeitet und über eine Schnittstelle an die Computereinrichtung 3 abgibt. Die Computereinrichtung 3 weist vorteilhaft eine Software zur Aufnahme, Speicherung und Darstellung dieser Daten auf der Anzeigeeinrichtung 4 auf.

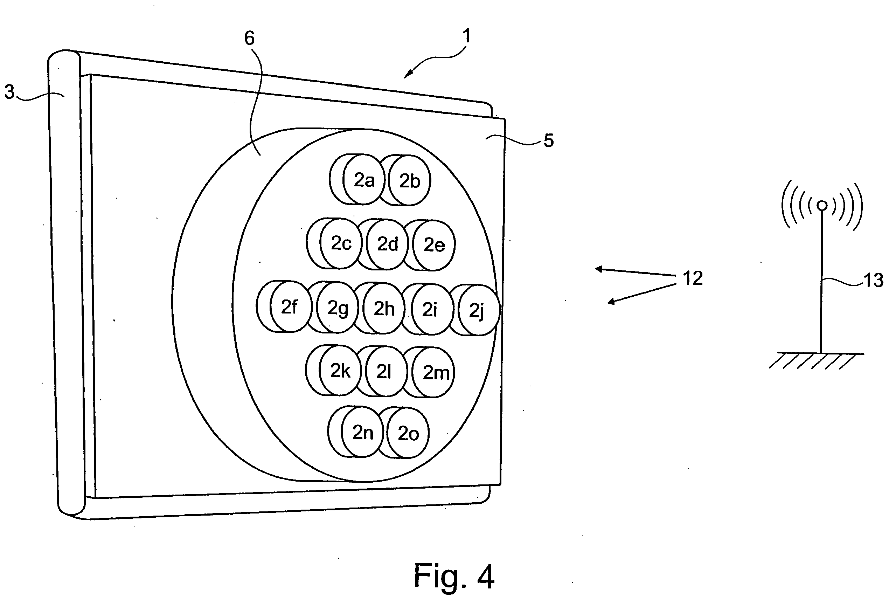

In der Fig. 4 ist eine weitere Ausführungsform des Diagnosegeräts 1 dargestellt, in der die Sensoren der Sensormatrix 2 mit Bezugszeichen 2a bis 2o durchnummeriert sind. Die Sensoren 2a bis 2o sind zusätzlich zur Erfassung eines Umgebungsfelds 12 vorgesehen. Als Umgebungsfeld 12 kommt z.B. eine in der Umgebung auftretenden Störstrahlung oder ein Rundfunksignal von einem Rundfunksender 13 in Frage. Das Umgebungsfeld 12 wird ebenfalls über die in dem Gehäuse 6 angeordnete Auswerteeinrichtung erfasst und ausgewertet und als Positionsinformation an die Computereinrichtung 3 übertragen. Die Computereinrichtung 3 ordnet bei der Erzeugung der Bildinformation aus den Spannungssignalen der Sensormatrix 2 die Positionsinformation der Bildinformation zu. Hierdurch ist die relative Lage der Sensormatrix 2 zum Körper des Menschen oder Tieres erfasst.

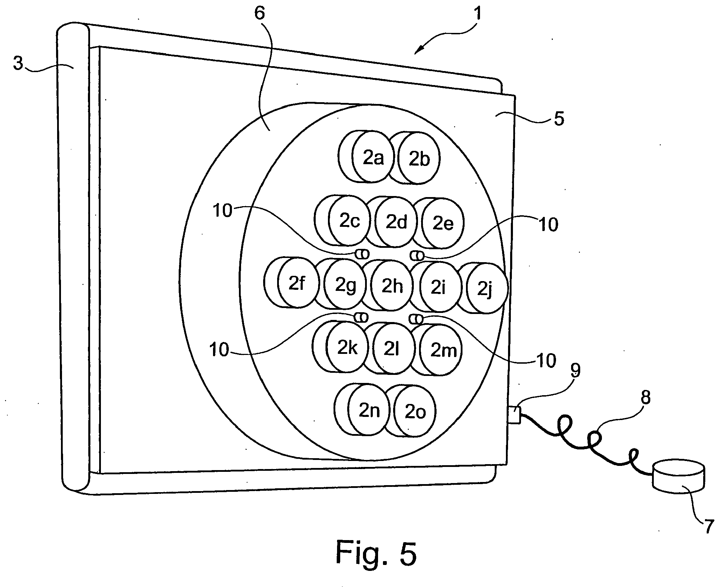

In der Fig. 5 ist eine weitere Ausführungsform des Diagnosegeräts 1 dargestellt. Das Diagnosegerät 1 ist über einen Anschlussstecker 9 und eine Leitung 8 mit einem Signalkoppler 7 verbunden. Der Signalkoppler 7 dient zur Einkopplung eines Positionssignals in dem Körper des Menschen oder Tieres. Der Signalkoppler wird an einer definierten Position am Körper befestigt. Diese Position ist derart vordefiniert, dass sie dem Diagnosegerät 1 bekannt ist. Das Diagnosegerät 1 erfasst die eingekoppelten Signale über die Sensormatrix 2 wiederum und nimmt daraus die Bestimmung der Positionsinformation vor. Des Weiteren ist das Diagnosegerät 1 mit weiteren Sensoren 10 versehen, die im Bereich der Sensormatrix 2 auf der Rückseite des Gehäuses 6 angeordnet sind. Die weiteren Sensoren 10 sind alternativ zur Erfassung des die Position der Sensormatrix relativ zum Körper repräsentierenden Signals vorgesehen, z. B. des von dem Signalkoppler 7 in den Körper eingekoppelten Signals. Die weiteren Sensoren 10 sind z. B. als Spulen ausgebildet, die zur Erfassung eines magnetischen Felds dienen.

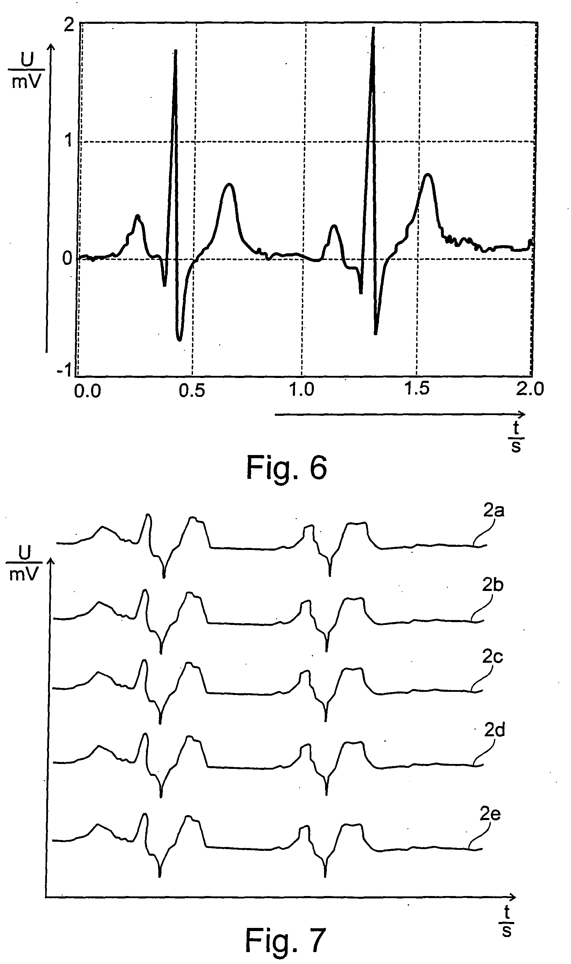

In der Fig. 6 ist ein übliches EKG-Signal dargestellt, wie es von bekannten EKG-Systemen aufgenommen wird. Über die Zeit t, gemessen in Sekunden s, ist ein Verlauf einer Spannung U, gemessen in mV (Millivolt), wiedergegeben.

Die Fig. 7 zeigt eine Mehrzahl von EKG-Signalen in einem Diagramm. Das erfindungsgemäße Diagnosegerät erlaubt aufgrund seiner Vielzahl von Sensoren der Sensormatrix 2 die Darstellung einer Vielzahl von EKG-Signalen. In der Fig. 7 ist lediglich beispielhaft eine Darstellung von Spannungsverläufen über die Zeit der von den Sensoren 2a, 2b, 2c, 2d und 2e gemessenen Spannungssignale wiedergegeben.

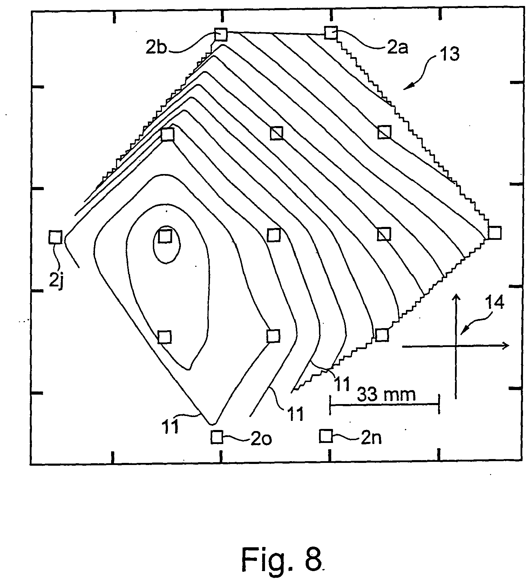

Die Fig. 8 zeigt eine ortsaufgelöste Darstellung der von den Sensoren 2a bis 2o aufgenommenen Spannungssignale, wie sie auf der Anzeigeeinrichtung 4 wiedergegeben werden kann. Die Spannungssignale sind zweidimensional ortsaufgelöst dargestellt und jeweils den realen Positionen der Sensoren 2a bis 2o auf der Rückseite des Gehäuses 6 zugeordnet. Die Positionen der Sensoren 2a bis 2o sind in der Darstellung durch quadratische Symbole wiedergegeben. Der Übersichtlichkeit halber sind lediglich die zu den Sensoren 2a, 2b, 2j, 2n und 2o zugehörigen Symbole mit entsprechenden Bezugszeichen versehen. Die Computereinrichtung 3 berechnet aus den Spannungssignalen der Sensoren 2a bis 2o Isopotentiallinien 11. Je nach Potentialhöhe, d. h. nach gemessener Spannung, sind die Flächen zwischen den Isopotentiallinien 11 in unterschiedlichen Graustufen oder mit unterschiedlichen Farben wiedergegeben.

In der Fig. 8 ist die Bildinformation 13 beispielhaft zusammen mit einer nach Art eines Koordinatenkreuzes dargestellten Positionsinformation 14 wiedergegeben. Die Positionsinformation 14 erlaubt dem Benutzer des Diagnosegeräts eine leichte Orientierung bezüglich der Position des Diagnosegeräts gegenüber dem Körper. Bei einer Verschiebung des Diagnosegeräts gegenüber dem Körper verschiebt sich entsprechend die Positionsinformation 14 in der Darstellung gemäß Fig. 8 bzw. auf der Anzeigeeinrichtung 4.

## Patentansprüche

1. Medizinisches Diagnosegerät (1) mit wenigstens einer Sensormatrix (2), die eine Mehrzahl von in bestimmter Weise verteilten Sensoren aufweist, zur Erfassung von elektrischen Spannungssignalen an einer Mehrzahl von Stellen an der Körperoberfläche eines Menschen oder Tieres, **dadurch gekennzeichnet, dass** das Diagnosegerät (1) zur Erfassung eines die Position der Sensormatrix (2) relativ zum Körper des Menschen oder Tieres repräsentierenden Signals (12) mittels der Sensoren der Sensormatrix und zur Bestimmung einer Positionsinformation (14) der Position der Sensormatrix (2) relativ zum Körper aus dem erfassten Signal (12) eingerichtet ist, wobei das Diagnosegerät (1) dazu eingerichtet ist, aus den Spannungssignalen eine ortsaufgelöste Bildinformation (13) zu bestimmen und der ortsaufgelösten Bildinformation (13) die Positionsinformation (14) zuzuordnen.

2. Medizinisches Diagnosegerät nach Anspruch 1, **dadurch gekennzeichnet, dass** das Diagnosegerät (1) zur Erfassung eines Elektrokardiogramms (EKG) oder eines Elektroenzephalogramms (EEG) eingerichtet ist.

3. Medizinisches Diagnosegerät nach wenigstens einem der vorhergehenden Ansprüche, **dadurch gekennzeichnet, dass** das Diagnosegerät (1) zur Erfassung eines elektromagnetischen Umgebungsfelds (12), insbesondere von Rundfunksignalen, und zur Auswertung des erfassten Felds zur Bestimmung der Position der Sensormatrix (2) relativ zum Körper des Menschen oder Tieres eingerichtet ist.

4. Medizinisches Diagnosegerät nach wenigstens einem der vorhergehenden Ansprüche, **dadurch gekennzeichnet, dass** das Diagnosegerät (1) einen weiteren Sensor (10) zur Erfassung des die Position der Sensormatrix (2) relativ zum Körper repräsentierenden Signals aufweist.

5. Medizinisches Diagnosegerät nach Anspruch 4, **dadurch gekennzeichnet, dass** der weitere Sensor (10) zur Erfassung eines magnetischen Felds, eines elektrischen Felds, eines elektromagnetischen Felds, eines Schallfelds oder von Röntgenstrahlung ausgebildet ist.

6. Medizinisches Diagnosegerät nach wenigstens einem der vorhergehenden Ansprüche, **dadurch gekennzeichnet, dass** ein Signalkoppler (7) zur Einkopplung eines Positionssignals in den Körper des Menschen oder Tieres vorgesehen ist.

7. Medizinisches Diagnosegerät nach Anspruch 6, **dadurch gekennzeichnet, dass** der Signalkoppler (7) zur Einkopplung eines magnetischen Felds, eines elektrischen Felds, eines elektromagnetischen Felds, eines Schallfelds oder von Röntgenstrahlung ausgebildet ist.

8. Medizinisches Diagnosegerät nach wenigstens einem der vorhergehenden Ansprüche, **dadurch gekennzeichnet, dass** die Sensormatrix (2) kapazitive Sensoren (2a bis 2o) aufweist.

9. Medizinisches Diagnosegerät nach wenigstens einem der vorhergehenden Ansprüche, **dadurch gekennzeichnet, dass** eine Anzeigeeinrichtung (4) vorgesehen ist, wobei das Diagnosegerät (1) dazu eingerichtet ist, eine Mehrzahl einzelner über die Sensormatrix (2) bestimmter Bildinformationen (13) anhand ihrer Positionsinformationen (14) miteinander zu korrelieren.

10. Medizinisches Diagnosegerät nach Anspruch 9, **dadurch gekennzeichnet, dass** auf der Anzeigeeinrichtung (4) eine Mehrzahl von Bildinformationen (13) aneinandergesetzt dargestellt ist.

11. Medizinisches Diagnosegerät nach Anspruch 9 oder 10, **dadurch gekennzeichnet, dass** auf der Anzeigeeinrichtung (4) eine Mehrzahl von Bildinformationen (13) zur Erhöhung der Auflösung der Darstellung überlagert dargestellt ist.

12. Medizinisches Diagnosegerät nach einem der vorhergehenden Ansprüche, **dadurch gekennzeichnet, dass** das Diagnosegerät als von einem Menschen tragbares Gerät ausgebildet ist, das für einen mobilen Einsatz eingerichtet ist.

13. Verfahren mit den Schritten
a) Erfassen von elektrischen Spannungssignalen an einer Mehrzahl von Stellen an der Körperoberfläche eines Menschen oder Tieres mittels einer Sensormatrix (2), die eine Mehrzahl von in bestimmter Weise verteilten Sensoren aufweist,
**gekennzeichnet durch**
b) Erfassen eines die Position der Sensormatrix (2) relativ zum Körper des Menschen oder Tieres repräsentierenden Signals (12) mittels der Sensoren der Sensormatrix,
c) Bestimmung einer Positionsinformation (14) der Position der Sensormatrix (2) relativ zum Körper aus dem erfassten Signal (12),
d) Bestimmung einer ortsaufgelösten Bildinformation (13) aus den Spannungssignalen und
e) Zuordnung der Positionsinformation (14) zu der Bildinformation (13).

## Claims

1. A medical diagnostic device (1) with at least one sensor matrix (2), which comprises a plurality of sensors distributed in a defined manner, for capturing electric voltage signals at a plurality of points on the body surface of a human or animal, wherein the diagnostic device (1) is designed to capture a signal (12) representing the position of the sensor matrix (2) relative to the body of the human or animal by means of the sensors in the sensor matrix and to determine position information (14), based upon the captured signal (12), in respect of the position of the sensor matrix (2) relative to the body, said diagnostic device (1) is designed to determine spatially resolved image information (13) from the voltage signals and to associate the spatially resolved image information (13) with the position information (14).

2. The medical diagnostic device as claimed in claim 1, **characterized in that** the diagnostic device (1) is designed to capture an electrocardiogram (EKG) or an electroencephalogram (EEG).

3. The medical diagnostic device as claimed in at least one of the preceding claims, **characterized in that** the diagnostic device (1) is designed to capture a surrounding electromagnetic field (12), more particularly radio signals, and to evaluate the captured field for determining the position of the sensor matrix (2) relative to the body of the human or animal.

4. The medical diagnostic device as claimed in at least one of the preceding claims, **characterized in that** the diagnostic device (1) has a further sensor (10) for capturing the signal representing the position of the sensor matrix (2) relative to the body.

5. The medical diagnostic device as claimed in claim 4, **characterized in that** the further sensor (10) is designed to capture a magnetic field, an electric field, an electromagnetic field, a sonic field or X-ray radiation.

6. The medical diagnostic device as claimed in at least one of the preceding claims, **characterized in that** provision is made for a signal coupler (7) for coupling a position signal into the body of the human or animal.

7. The medical diagnostic device as claimed in claim 6, **characterized in that** the signal coupler (7) is designed to couple in a magnetic field, an electric field, an electromagnetic field, a sonic field or X-ray radiation.

8. The medical diagnostic device as claimed in at least one of the preceding claims, **characterized in that** the sensor matrix (2) has capacitive sensors (2a to 2o).

9. The medical diagnostic device as claimed in at least one of the preceding claims, **characterized in that** provision is made for a display apparatus (4), wherein the diagnostic device (1) is designed to correlate a plurality of individual items of image information (13), determined by the sensor matrix (2), to one another on the basis of their position information (14).

10. The medical diagnostic device as claimed in claim 9, **characterized in that** a plurality of items of image information (13) are displayed side-by-side on the display apparatus (4).

11. The medical diagnostic device as claimed in claim 9 or 10, **characterized in that** a plurality of items of image information (13) are displayed superposed on one another on the display apparatus (4) in order to increase the resolution of the display.

12. The medical diagnostic device as claimed in one of the preceding claims, **characterized in that** the diagnostic device is designed as a device that can be carried by a human and is embodied for mobile use.

13. A method comprising the steps of:
a) capturing electric voltage signals at a plurality of points on the body surface of a human or animal by means of a sensor matrix (2), which comprises a plurality of sensors distributed in a certain manner, **characterized by**
b) capturing a signal (12) representing the position of the sensor matrix (2) relative to the body of the human or animal by means of the sensors in the sensor matrix,
c) determining position information (14) in respect of the position of the sensor matrix (2) relative to the body from the captured signal (12),
d) determining spatially resolved image information (13) from the voltage signals and
e) associating the position information (14) with the image information (13).

## Revendications

1. Instrument de diagnostic médical (1), avec au moins une matrice de capteurs (2), qui comporte une pluralité de capteurs distribués d'une certaine manière, pour détecter des signaux de tension électrique sur une pluralité d'endroits sur la surface du corps d'un humain ou d'un animal, **caractérisé en ce que** l'instrument de diagnostic (1) est aménagé pour détecter un signal (12) représentant la position de la matrice de capteurs (2) par rapport au corps de l'humain ou de l'animal au moyen des capteurs de la matrice de capteurs et pour déterminer à partir du signal (12) détecté une information de position (14) sur la position de la matrice de capteurs (2) par rapport au corps, l'instrument de diagnostic (1) étant aménagé pour déterminer à partir des signaux de tension une information d'image (13) à résolution locale et pour associer à l'information d'image (13) à résolution locale l'information de position (14).

2. Instrument de diagnostic médical selon la revendication 1, **caractérisé en ce que** l'instrument de diagnostic (1) est aménagé pour détecter un électrocardiogramme (ECG) ou un électroencéphalogramme (EEG).

3. Instrument de diagnostic médical selon au moins l'une quelconque des revendications précédentes, **caractérisé en ce que** l'instrument de diagnostic (1) est aménagé pour détecter un champ électromagnétique (12) environnant, notamment de signaux radio et pour évaluer le champ détecté, afin de déterminer la position de la matrice de capteurs (2) par rapport au corps de l'humain ou de l'animal.

4. Instrument de diagnostic médical selon au moins l'une quelconque des revendications précédentes, **caractérisé en ce que** l'instrument de diagnostic (1) comporte un capteur (10) supplémentaire pour détecter le signal représentant la position de la matrice de capteurs (2) par rapport au corps.

5. Instrument de diagnostic médical selon la revendication 4, **caractérisé en ce que** le capteur (10) supplémentaire est conçu pour détecter un champ magnétique, un champ électrique, un champ électromagnétique, un champ acoustique ou des rayons X.

6. Instrument de diagnostic médical selon au moins l'une quelconque des revendications précédentes, **caractérisé en ce qu'**un coupleur de signaux (7) est prévu pou injecter un signal de position dans le corps de l'humain ou de l'animal.

7. Instrument de diagnostic médical selon la revendication 6, **caractérisé en ce que** le coupleur de signaux (7) est prévu pou injecter un champ magnétique, un champ électrique, un champ électromagnétique, un champ acoustique ou des rayons X.

8. Instrument de diagnostic médical selon au moins l'une quelconque des revendications précédentes, **caractérisé en ce que** la matrice de capteurs (2) comporte des capteurs (2a à 2o) capacitifs.

9. Instrument de diagnostic médical selon au moins l'une quelconque des revendications précédentes, **caractérisé en ce qu'**un dispositif d'affichage (4) est prévu, l'instrument de diagnostic (1) étant aménagé pour mettre en corrélation mutuelle une pluralité d'informations d'image (13) déterminées par l'intermédiaire de la matrice de capteurs (2) à l'aide de ses informations de position (14).

10. Instrument de diagnostic médical selon la revendication 9, **caractérisé en ce que** sur le dispositif d'affichage (4) est représentée une pluralité d'informations d'images (13) mises bout à bout.

11. Instrument de diagnostic médical selon la revendication 9 ou 10, **caractérisé en ce que** pour augmenter la résolution de la représentation, sur le dispositif d'affichage (4) une pluralité d'informations d'images (13) est représentée en superposition.

12. Instrument de diagnostic médical selon l'une quelconque des revendications précédentes, **caractérisé en ce que** l'instrument de diagnostic est conçu en tant qu'un instrument portable par un individu, qui est aménagé pour une utilisation mobile.

13. Procédé comportant les étapes
a) de la détection de signaux de tension électriques sur une pluralité d'endroits sur la surface du corps d'un humain ou d'un animal au moyen d'une matrice de capteurs (2), qui comporte une pluralité de capteurs distribués d'une certaine manière **caractérisé par**
b) la détection d'un signal (12) représentant la position de la matrice de capteurs (2) par rapport au corps de l'humain ou de l'animal au moyen des capteurs de la matrice de capteurs,
c) la détermination d'une information de position (14) sur la position de la matrice de capteurs (2) par rapport au corps, à partir du signal (12) détecté,
d) la détermination d'une information d'image (13) à résolution locale à partir des signaux de tension et
e) l'association de l'information de position (14) à l'information d'image (13).
